# EUROPEAN PATENT APPLICATION

(11) **EP 4 760 256 A1**
(43) Date of publication of application: **17.06.2026**
(21) Application number: 25222450.6
(22) Date of filing: 11.12.2025
(51) Int. Cl.: G01N 33/536, G01N 33/543

(54) **METHOD OF MEASURING TARGET SUBSTANCE, AND REAGENT THEREFOR**

(30) Priority: 13.12.2024 JP 2024218551; 04.11.2025 JP 2025185663
(71) Applicant: Canon Kabushiki Kaisha, Tokyo, 146-8501 (JP)
(72) Inventor: SHIMANO, Tsutomu, Tokyo (JP); ISHIOKA, Shuhei, Tokyo (JP); WAKISAKA, Risa, Tokyo (JP); KANAZAKI, Kengo, Tokyo (JP); OONUKI, Sayaka, Tokyo (JP); FUNAYAMA, Kiyotake, Tokyo (JP)
(74) Representative: WESER & Kollegen Patentanwälte PartmbB

(57) **Abstract**

Provided are a measurement method for a target substance using immunoturbidimetry and a reagent therefor. The method is for measuring a target substance in a specimen, the target substance being a protein having a molecular weight of 150 to 500 kDa, incorporated into the specimen at a concentration of 0.8 to 2.0 ng/mL. The method includes: mixing the specimen and a first reagent to provide a first solution; mixing the first solution and a second reagent, including a particle immobilized a ligand that binds to the target substance, to provide a second solution; and measuring the absorbance of the second solution. The concentration of the specimen in the second solution is 4.0 to 30.0 vol%, the mass ratio of the particle to the specimen in the second solution is 1:500 to 1:50, and an amount of change in absorbance per 1.0 ng/mL of the target substance is 0.0012 to 0.0100.

## Description

### TECHNICAL FIELD

The present disclosure relates to a method of measuring a target substance, and a reagent therefor.

### BACKGROUND

There is given immunoturbidimetry using a particle as a simple and rapid immunological testing method. In this method, a dispersion of a particle to which a ligand having affinity to a target substance is bonded, and a specimen that may contain the target substance are mixed. At this time, the particle causes an aggregation reaction in accordance with the amount of the target substance in the specimen, and hence the target substance can be qualitatively or quantitatively determined by optically detecting the aggregation reaction as a variation in, for example, scattered light intensity, transmitted light intensity, or absorbance.

Many immuno-testing items in clinical tests have diagnostically important points in a region where the target substance is present at a low concentration, and it is required that the target substance can be quantitatively determined even at a low concentration. In particular, when a protein having a large molecular weight is used as the target substance, the number of molecules of the target substance in a unit specimen amount reduces, and the reduction makes the aggregation reaction less likely to occur. For this reason, quantitative determination is more difficult.

To address such problem, Japanese Patent Laid-Open No. 2006-71297 discloses a measurement method by immunoturbidimetry in which the amount of change in absorbance is increased by using a particle having a large particle diameter. Japanese Patent Laid-Open No. 2017-83440 discloses a measurement method by immunoturbidimetry for ferritin measurement, in which an antigen-antibody reaction is performed in the presence of a buffer solution having a concentration of 0.19 mol/L or more. Ferritin is a protein having a molecular weight of 445 kDa, and hence can be said to be a protein having a large molecular weight. In addition, in "Fundamental Performance of 'Iatro Ferritin' Reagent for Measurement of Ferritin on a Multi-purpose Automated Analyzer," JJCLA Vol. 31, No. 5, 2006, there is a disclosure of an immunoturbidmetric reagent for ferritin measurement having a minimum detection sensitivity of 4.8 ng/mL. In addition, in "Examination of 'LZ Test Eiken FER' Reagent for Measurement of Ferritin for a Multi-purpose Automated Analyzer," Instruments and Reagents 26(4), 2003, there is a disclosure of an immunoturbidmetric reagent for ferritin measurement having a minimum detection sensitivity of 3.52 ng/mL. Further, in "Fundamental Performance Analysis of a Reagent for Measurement of Ferritin using Fully Automated Chemiluminescent Enzyme Immunoassay System LUMIPULSE L2400," Instruments and Reagents 39(4), 2016, there is a disclosure of a ferritin measuring reagent using a chemiluminescent enzyme immunoassay, and there is a disclosure that its detection sensitivity (referred to as "quantification limit" in the literature) is 0.2 ng/mL or less. The chemiluminescent enzyme immunoassay is a method in which an enzyme-labeled antibody is caused to secondarily react with an antigen after the reaction of the antigen with an antibody immobilized on a solid phase, and then a chemiluminescent substrate is added to measure the luminescence intensity of the enzyme-labeled antibody.

As disclosed in Japanese Patent Laid-Open No. 2006-71297, it is a known idea that the occurrence of a large change in absorbance is effective in improving sensitivity in immunoturbidimetry using a particle.

### SUMMARY

However, in the measurement method disclosed in Japanese Patent Laid-Open No. 2006-71297, C-reactive protein (CRP) is measured as the target substance at a concentration of 0.25 µg/dL or more, and there is no disclosure of the measurement at a lower concentration. CRP is a protein having a molecular weight of 105 kDa. The measurement method by immunoturbidimetry using a particle disclosed in Japanese Patent Laid-Open No. 2017-83440 involves performing quantitative determination in a ferritin concentration range of 103 ng/mL or more, and there is no disclosure of an attempt to quantitatively determine ferritin having a concentration of less than 103 ng/mL.

In addition, the minimum detection sensitivities of the immunoturbidmetric reagents for ferritin measurement disclosed in "Fundamental Performance of 'Iatro Ferritin' Reagent for Measurement of Ferritin on a Multi-purpose Automated Analyzer," JJCLA Vol. 31, No. 5, 2006, and "Examination of 'LZ Test Eiken FER' Reagent for Measurement of Ferritin for a Multi-purpose Automated Analyzer," Instruments and Reagents 26(4), 2003, are 4.8 ng/mL and 3.52 ng/mL, respectively. Accordingly, ferritin having a concentration of less than 3.52 ng/mL cannot be quantitatively determined. In addition, a change in absorbance of the immunoturbidmetric reagent for ferritin measurement disclosed in "Fundamental Performance of 'Iatro Ferritin' Reagent for Measurement of Ferritin on a Multi-purpose Automated Analyzer," JJCLA Vol. 31, No. 5, 2006, is read to be about 0.0010 or less for a specimen having a ferritin concentration of 1.19 ng/mL. In addition, a change in absorbance of the immunoturbidmetric reagent for ferritin measurement disclosed in "Examination of 'LZ Test Eiken FER' Reagent for Measurement of Ferritin for a Multi-purpose Automated Analyzer," Instruments and Reagents 26(4), 2003, is read to be about 0.0005 or less for a specimen having a ferritin concentration of 2 ng/mL.

As described in those literatures, the minimum detection sensitivity of ferritin is 3.52 ng/mL or more for any of the immunoturbidmetric reagents, and there is no disclosure of an attempt to quantitatively determine ferritin having an extremely low concentration such as 2.0 ng/mL or less, or any suggestive description thereof. In addition, in order to detect ferritin having an extremely low concentration such as 2.0 ng/mL or less, a chemiluminescent method such as the chemiluminescent enzyme immunoassay disclosed in "Fundamental Performance Analysis of a Reagent for Measurement of Ferritin using Fully Automated Chemiluminescent Enzyme Immunoassay System LUMIPULSE L2400," Instruments and Reagents 39(4), 2016, is generally used, and there has been no attempt to achieve the detection using immunoturbidimetry.

Immunoturbidimetry is a simple and rapid method as compared to the chemiluminescent method, and the achievement of target substance measurement by the immunoturbidimetry is extremely useful. However, the measurement principle of the immunoturbidimetry differs significantly from that of the chemiluminescent method. In particular, in means for achieving low concentration sensitivity, a technique used in the chemiluminescent method is rarely effective for the immunoturbidimetry.

It is assumed from the foregoing that there is no disclosure of a method of quantitatively determining a protein having a large molecular weight as a target substance in an extremely low concentration range such as 2.0 ng/mL or less by simple and rapid immunoturbidimetry, and attempts themselves are not actively made.

The present disclosure has been made in view of such background art and problems. The present disclosure is directed to providing a measurement method for quantitatively determining a concentration of 2.0 ng/mL or less, with a protein having a molecular weight of 150 kDa or more as a target substance, by immunoturbidimetry, and a reagent therefor.

The present disclosure has found that sufficient measurement reproducibility is obtained only by satisfying a specific measurement method when a change in absorbance caused by particle aggregation is set to a certain level or more at the time of the use of a specimen having a target substance concentration of 2.0 ng/mL or less, and the present disclosure has completed a measurement method that provides excellent quantitative capability.

That is, the present disclosure in its first aspect provides a measurement method for measuring a target substance in a specimen, the target substance being a protein having a molecular weight of 150 kDa or more and 500 kDa or less, which is incorporated into the specimen at a concentration of 0.8 ng/mL or more and 2.0 ng/mL or less, the measurement method including: a first mixing step of mixing the specimen and a first reagent to provide a first mixed solution; a second mixing step of mixing the first mixed solution and a second reagent, which includes a particle having immobilized thereon a ligand that specifically binds to the target substance, to provide a second mixed solution; and a measurement step of measuring an absorbance of the second mixed solution, wherein a concentration of the specimen in the second mixed solution is 4.0 vol% or more and 30.0 vol% or less, wherein a mass ratio of the particle to the specimen in the second mixed solution is 1:500 or more and 1:50 or less, and wherein the measurement method includes measuring the specimen in which an amount of change in absorbance per 1.0 ng/mL of the target substance in the measurement step is 0.0012 or more and less than 0.0100 as specified in claim 1. Optional features are specified in claims 2 to 21.

The foregoing relates to a measurement method in which a difference ΔOD1-ΔOD0 between a change in absorbance ΔOD1 when the specimen is a specimen 1 including the target substance at a concentration of 1.0 ng/mL or more and 2.0 ng/mL or less, and a change in absorbance ΔOD0 when the specimen is a specimen 0 including the target substance at a concentration of 0.0 ng/mL or more and less than 0.5 ng/mL is 0.0012 or more and less than 0.0100.

The present disclosure in its second aspect provides a reagent for measuring a target substance in a specimen, wherein the target substance is a protein having a molecular weight of 150 kDa or more and 500 kDa or less, which is incorporated into the specimen at a concentration of 0.8 ng/mL or more and 2.0 ng/mL or less, wherein the reagent includes a first reagent and a second reagent including a particle having immobilized thereon a ligand that specifically binds to the target substance, and wherein in a case where ΔOD1a represents a difference "absorbance B-absorbance A" between an absorbance A at a time point of 35 seconds or more and 45 seconds or less after addition of the second reagent and stirring, and an absorbance B at a time point of 290 seconds or more and 300 seconds or less thereafter when a first mixed solution is prepared by mixing 15 µL of the specimen, which includes 1.0 ng/mL of the target substance, 50 mM of N-2-hydroxyethylpiperazine-N'-2-ethanesulfonic acid, and ultrapure water, with 60 µL of the first reagent under a condition of 37°C, and 30 µL of the second reagent is added to the first mixed solution and the mixture is stirred at a time point after a lapse of 280 seconds or more and 300 seconds or less from the mixing, and ΔOD0a represents a difference "absorbance D-absorbance C" between an absorbance C at a time point of 35 seconds or more and 45 seconds or less after addition of the second reagent and stirring, and an absorbance D at a time point of 290 seconds or more and 300 seconds or less thereafter when the first mixed solution is prepared by mixing 15 µL of the specimen, which includes 50 mM of N-2-hydroxyethylpiperazine-N'-2-ethanesulfonic acid and ultrapure water, with 60 µL of the first reagent under the condition of 37°C, and 30 µL of the second reagent is added to the first mixed solution and the mixture is stirred at a time point after a lapse of 280 seconds or more and 300 seconds or less from the mixing, a difference "ΔOD1a-ΔOD0a" is 0.0012 or more when a measurement wavelength of each of the absorbances is 572 nm as specified in claim 22.

According to the present disclosure, the measurement method for quantitatively determining a concentration of 2.0 ng/mL or less with a protein having a molecular weight of 150 kDa or more as a target substance by simple and rapid immunoturbidimetry, and the reagent therefor can be provided.

Features of the present disclosure will become apparent from the following description of embodiments. The following description of embodiments is described by way of example.

### DESCRIPTION OF THE EMBODIMENTS

Embodiments of the present disclosure are described in detail below. However, the technical scope of the present disclosure is not limited to the embodiments. A particle having immobilized thereon a ligand that specifically binds to a target substance is hereinafter also referred to as "affinity particle," and a particle before the ligand is immobilized thereon is also referred to as "pre-sensitization particle."

The inventors have made investigations on a measurement method in which a change in absorbance increases even when the concentration of the target substance is 2.0 ng/mL or less. As a result of the intensive investigations, the inventors have found that when the change in absorbance is 0.0012 or more, detection sensitivity is significantly improved only in the case where a specific measurement condition is satisfied, and the inventors have been able to recognize measurement reproducibility. This tendency is different from that of a state in which the change in absorbance is less than 0.0012, and the difference suggests that the tendency is a phenomenon unique to a change in absorbance of 0.0012 or more. The inventors have assumed that this is because of a new effect resulting from particle aggregation caused by the target substance, the effect being significantly larger than an influence of measurement error that inevitably occurs in absorbance measurement.

As a result of further investigations of this finding, the present disclosure has found a measurement method that can achieve a detection sensitivity of 2.0 ng/mL or less even though the measurement method is immunoturbidimetry. It can be said that the foregoing cannot be achieved until the inventors have made intensive investigations on such related art as disclosed in Japanese Patent Laid-Open No. 2006-71297 and Japanese Patent Laid-Open No. 2017-83440, and "Fundamental Performance of 'Iatro Ferritin' Reagent for Measurement of Ferritin on a Multi-purpose Automated Analyzer," JJCLA Vol. 31, No. 5, 2006, and "Examination of 'LZ Test Eiken FER' Reagent for Measurement of Ferritin for a Multi-purpose Automated Analyzer," Instruments and Reagents 26(4), 2003.

That is, the present disclosure relates to a measurement method for measuring a target substance in a specimen, the target substance being a protein having a molecular weight of 150 kDa or more and 500 kDa or less, which is incorporated into the specimen at a concentration of 0.8 ng/mL or more and 2.0 ng/mL or less, the measurement method including: a first mixing step of mixing the specimen and a first reagent to provide a first mixed solution; a second mixing step of mixing the first mixed solution and a second reagent, which includes a particle having immobilized thereon a ligand that specifically binds to the target substance, to provide a second mixed solution; and a measurement step of measuring the absorbance of the second mixed solution, wherein a concentration of the specimen in the second mixed solution is 4.0 vol% or more and 30.0 vol% or less, wherein a mass ratio of the particle to the specimen in the second mixed solution is 1:500 or more and 1:50 or less, and wherein the measurement method includes measuring the specimen in which an amount of change in absorbance per 1.0 ng/mL of the target substance in the measurement step is 0.0012 or more and less than 0.0100.

In this embodiment, the mixing of the specimen and the first reagent may refer to the addition of one of the specimen or the first reagent to the other, or may refer to the addition of the specimen and the first reagent to each other. The same applies to any other mixing in the present specification. In addition, the phrase "from the mixing" in this embodiment means "from the time of the completion of stirring when the entire amounts of two kinds of liquids are mixed, and the stirring is performed to sufficiently mix the two kinds of liquids." In addition, a time period from the time of the start of the mixing of the two kinds of liquids to the time of the completion of the above-mentioned stirring is 5 seconds or less. The stirring may be performed with a stirring bar, by irradiation with ultrasonic waves, or by tapping. For example, the fact that the absorbance 45 seconds after the mixing is 0.90 or more and 2.00 or less means that an absorbance measured at a time of 40 seconds from the time of the completion of the above-mentioned stirring is 0.90 or more and 2.00 or less.

When the concentration of the specimen in the second mixed solution is less than 4.0 vol%, sufficient detection sensitivity is not obtained. When the concentration is more than 30.0 vol%, particle aggregation is affected by a component other than the target substance in the specimen. Thus, measurement accuracy deteriorates and hence sufficient detection sensitivity is not obtained. In addition, the measurement accuracy also deteriorates when the concentration of the target substance in the specimen is high. The concentration of the specimen in the second mixed solution is more preferably 7.0 vol% or more and 30.0 vol% or less, still more preferably 10.0 vol% or more and 20.0 vol% or less.

Sufficient measurement accuracy is obtained only when the mass ratio of the particle to the specimen in the second mixed solution is 1:500 or more and 1:50 or less even in the case where the amount of change in absorbance per 1.0 ng/mL of the target substance is 0.0012 or more. This is probably because this ratio allows a component other than the target substance in the specimen to act without bias toward each particle. Accordingly, in the case where this ratio is not satisfied, the measurement accuracy is significantly reduced when the amount of change in absorbance per 1.0 ng/mL of the target substance is 0.0012 or more. Accordingly, sufficient detection sensitivity is not obtained. The mass ratio of the particle to the specimen in the second mixed solution is more preferably 1:500 or more and 1:80 or less, still more preferably 1:500 or more and 1:100 or less.

When the amount of change in absorbance per 1.0 ng/mL of the target substance is less than 0.0012, the measurement accuracy decreases simultaneously with an increase in ΔOD, and hence sufficient detection sensitivity is not obtained. A small amount of change in absorbance per 1.0 ng/mL of a protein means that the degree of aggregation of the particles specifically binding to the target substance is small. Accordingly, it is conceivable that the measurement accuracy of the absorbance measurement itself greatly influences the result, and hence sufficient detection sensitivity is not obtained. In addition, it is conceivable that the ratio of the aggregated particles is low and hence an aggregated state is non-uniform. Accordingly, it can be assumed that the state is non-uniform. The inventors have assumed that the measurement accuracy is reduced by increasing the ΔOD in this state. When the amount of change in absorbance per 1.0 ng/mL of the target substance is 0.0012 or more, the degree of aggregation of the particles specifically binding to the target substance may be larger than the influence of the accuracy of the absorbance measurement itself. In addition, the ratio of the aggregated particles is high and hence the aggregated state is uniform. Accordingly, sufficient detection sensitivity may be obtained. When the amount of change in absorbance per 1.0 ng/mL of the target substance is 0.0100 or more, the change in absorbance is so large that sufficient measurement reproducibility is not obtained. Accordingly, sufficient detection sensitivity, in particular, the quantitative capability of the target substance is not obtained. The amount of change in absorbance per 1.0 ng/mL of the target substance is more preferably 0.0015 or more and less than 0.0100, still more preferably 0.0020 or more and less than 0.0100.

### <Detection Method and Measurement Method>

A measurement method to be used in the present disclosure is immunoturbidimetry using a particle. The immunoturbidimetry using a particle is a method of optically detecting inter-particle aggregation that occurs when the affinity particle of the present disclosure and the specimen are mixed. In the present disclosure, an absorbance is used as a method of detecting the optical change. There is no limitation on an instrument to be used in the measurement, and any optical instrument capable of detecting the absorbance may be used. Of those, a general-purpose automatic analyzer, in which the dispensing amount of the specimen or the reagent, a mixing time, a measurement wavelength, and the like are easy to control, is preferably used.

An example of a measurement method using the automatic analyzer is described below. First, 8 µL to 25 µL of the specimen is dispensed into a reaction container. Next, as the first mixing step, 50 µL to 150 µL of the first reagent is dispensed into the same reaction container. After the mixture has been stirred, the temperature of the container is adjusted to a predetermined temperature, and the container is kept warm for from 180 seconds to 600 seconds. The temperature at this time preferably falls within the range of from 20°C to 50°C.

After that, as the second mixing step, 10 µL to 150 µL of the second reagent is dispensed into the same reaction container, and after stirring, a reaction is performed for from 180 seconds to 600 seconds. At this time, it is preferred to start the second mixing step after 280 seconds or more have elapsed from the mixing in the first mixing step. After the lapse of 280 seconds from the mixing in the first mixing step, the specimen and the first reagent are uniformly mixed, and hence the measurement accuracy is improved when the amount of change in absorbance per 1.0 ng/mL of the target substance is 0.0012 or more. In addition, the reaction time of the second mixing step is more preferably 200 seconds or more and 600 seconds or less.

Further, the absorbance of the second mixed solution is measured, and the amount of change in absorbance is calculated. At this time, the absorbance 40 seconds after the mixing in the second mixing step is preferably 0.90 or more and 2.00 or less. This is because the absorbance 40 seconds after the mixing in the second mixing step indicates the initial absorbance before the aggregation reaction of the particles proceeds, and when the value falls within the above-mentioned range, sufficient measurement accuracy is obtained even in the case where the concentration of the target substance is 2.0 ng/mL or less. In addition, the measurement wavelength of the absorbance preferably includes 500 nm or more and 750 nm or less. This is because, when the measurement wavelength includes a wavelength that falls within the above-mentioned range, a sufficient amount of change in absorbance is easily obtained and measurement accuracy is easily obtained even in the case where the concentration is 2.0 ng/mL or less.

The concentration of the affinity particle in the second mixed solution is preferably 0.02 mass/vol% or more and 0.10 mass/vol% or less. When the concentration is 0.02 mass/vol% or more and 0.10 mass/vol% or less, the ratio of the particles to be aggregated is appropriate in the case where the concentration of the target substance is 2.0 ng/mL or less, and hence sufficient measurement accuracy is obtained. In addition, a pH in the second mixed solution is preferably 5.0 or more and 11.0 or less.

The viscosity of the second mixed solution is preferably 0.95 mPa·s or more and 1.40 mPa·s or less. When the viscosity falls within the above-mentioned range, the rate of particle aggregation is suppressed, and hence higher measurement accuracy is obtained in the case where the amount of change in absorbance per 1.0 ng/mL of the target substance is 0.0012 or more. In addition, excellent measurement accuracy is obtained even when the concentration of the target substance is high.

### <Specimen and Target Substance>

The specimen that may be used in the present disclosure is not particularly limited as long as the specimen contains the target substance, and examples thereof include blood, serum, and plasma.

The target substance of the present disclosure is a protein having a molecular weight of 150 kDa or more and 500 kDa or less. Examples of such target substance include ferritin, sialylated carbohydrate antigen KL-6, carcinoembryonic antigen (CEA), and carbohydrate antigen 15-3, but the target substance is not limited thereto, and only needs to be a protein having a molecular weight that falls within the above-mentioned range. In the measurement method of this embodiment, the target substance is more preferably a protein having a molecular weight of 300 kDa or more and 600 kDa or less, still more preferably ferritin among others. Ferritin is a protein in which 24 subunits of two kinds are assembled, and has a plurality of reaction points with an antibody. Accordingly, it is conceivable that a plurality of particles may be involved to form non-uniform aggregation. According to the measurement method of the present disclosure, the number of binding ferritin molecules between the respective affinity particles can be uniformized, and hence the aggregation reaction is easily controlled to be uniform, and high measurement accuracy is obtained. The target substance of the present disclosure is preferably a substance having a plurality of reaction sites for an antibody (ligand) that specifically binds to the target substance, and ferritin is also preferable from this viewpoint. The target substance is preferably incorporated into the specimen at a concentration of 0.8 ng/mL or more and 2.0 ng/mL or less, more preferably incorporated at a concentration of 1.0 ng/mL or more and 1.5 ng/mL or less.

### <First Reagent and Second Reagent>

The reagent to be used in the measurement method of the present disclosure includes the first reagent and the second reagent including the affinity particle having immobilized thereon a ligand that specifically binds to the target substance.

In this embodiment, the reagent is a reagent in which in the case where ΔOD1a represents a difference "absorbance B-absorbance A" between an absorbance A at a time point of 35 seconds or more and 45 seconds or less after the addition of the second reagent and stirring, and an absorbance B at a time point of 290 seconds or more and 300 seconds or less thereafter when a first mixed solution is prepared by mixing 15 µL of the specimen, which includes 1.0 ng/mL of the target substance, 50 mM of N-2-hydroxyethylpiperazine-N'-2-ethanesulfonic acid, and ultrapure water, with 60 µL of the first reagent under the condition of 37°C, and 30 µL of the second reagent is added to the first mixed solution and the mixture is stirred at a time point after the lapse of 280 seconds or more and 300 seconds or less from the mixing, and ΔOD0a represents a difference "absorbance D-absorbance C" between an absorbance C at a time point of 35 seconds or more and 45 seconds or less after the addition of the second reagent and stirring, and an absorbance D at a time point of 290 seconds or more and 300 seconds or less thereafter when the first mixed solution is prepared by mixing 15 µL of the specimen, which includes 50 mM of N-2-hydroxyethylpiperazine-N'-2-ethanesulfonic acid and ultrapure water, with 60 µL of the first reagent under the condition of 37°C, and 30 µL of the second reagent is added to the first mixed solution and the mixture is stirred at a time point after the lapse of 280 seconds or more and 300 seconds or less from the mixing, a difference "ΔOD1a-ΔOD0a" is 0.0012 or more when the measurement wavelength of each of the absorbances is 572 nm.

The first reagent is used for the role of diluting the specimen or subjecting the specimen to prevent non-specific reaction. Accordingly, the first reagent may include such a buffering agent, sugar, surfactant, sensitizer, or non-specific reaction inhibitor as described later. The surface tension of the first reagent is preferably 20 mN/m or more and 50 mN/m or less. When the surface tension falls within the above-mentioned range, the affinity particle and the specimen are uniformly mixed. As a result, the action of the specimen component on the affinity particle is uniformized, and hence higher measurement accuracy is obtained when the amount of change in absorbance per 1.0 ng/mL of the target substance is 0.0012 or more.

The electrical conductivity of the first reagent is preferably 0.5 mS/cm or more and 70.0 mS/cm or less, more preferably 10.0 mS/cm or more and 70.0 mS/cm or less. When the electrical conductivity falls within the above-mentioned ranges, an electrostatic repulsion force between the affinity particles is maintained, and hence an interaction caused by an anionic specimen component, in particular, is uniformized for each affinity particle, and more excellent measurement accuracy is obtained in the case where the amount of change in absorbance per 1.0 ng/mL of the target substance is 0.0012 or more. The electrical conductivity of the first reagent is more preferably 0.5 mS/cm or more and 65.0 mS/cm or less.

The pH of each of the first reagent and the second reagent is preferably 5.0 or more and 11.0 or less. When the pH falls within the above-mentioned range, the dispersed state of the affinity particle and a mixed state at the time of the mixing of the specimen with the particle can be uniformized. Accordingly, more excellent measurement accuracy is obtained when the amount of change in absorbance per 1.0 ng/mL of the protein is 0.0012 or more. The pHs of the first reagent and the second reagent may be values different from each other.

The first reagent and the second reagent each preferably include a buffering agent (buffer). The kind of the buffer is not particularly limited, and only needs to be a substance from which a buffering capacity is obtained. For example, acetate-, citrate-, phosphate,- Tris-, glycine-, borate-, and carbonate-based buffering agents, and Good's buffering agents, such as MES, Bis-Tris, ADA, PIPES, ACES, MOPSO, BES, MOPS, TES, HEPES, TAPSO, POPSO, HEPSO, EPPS, Tricine, Bicine, TAPS, CHES, and CAPS, are suitably used. The buffering agents may be used alone or in combination thereof. In addition, the buffering agents to be used in the first reagent and the second reagent may be identical to or different from each other.

It is preferred that the first reagent and the second reagent each further include a sugar or a sugar alcohol. When the first reagent and the second reagent each include a sugar, the hydration of the surface of the affinity particle or the specimen component is promoted to reduce an interaction between the affinity particle and the specimen component, and hence the measurement accuracy is improved. In addition, a particle mobility rate is controlled by an increase in viscosity of a liquid, and hence high measurement accuracy is obtained when the time period of the second mixing step is 300 seconds or less. Examples of such sugar and sugar alcohol include, but not limited to: monosaccharides, such as glucose and fructose; disaccharides, such as sucrose, lactose, maltose, cellobiose, and trehalose; or oligosaccharides, such as maltotriose and dextran; and sugar alcohols, such as erythritol, mannitol, sorbitol, and xylitol. The sugars and the sugar alcohols may each be used alone or in combination thereof.

It is preferred that the first reagent and the second reagent each further include a surfactant. A known nonionic surfactant, anionic surfactant, cationic surfactant, or amphoteric surfactant may be used as the surfactant. The first reagent and the second reagent each preferably include one or more of a sorbitan fatty acid ester, a polyoxyethylene alkyl ether, and a polyoxyethylene phenyl ether out of those surfactants. Those surfactants each have high hydrophilicity, and high solubilizing and stabilizing effects on the specimen component. Accordingly, the interaction between the affinity particle and the specimen component is reduced, and hence excellent measurement accuracy is obtained even when the concentration of the target substance is high. The concentration of the surfactant is preferably 0.001 mass% or more and 0.200 mass% or less in the second mixed solution.

The first reagent may further include a sensitizer. Examples of the sensitizer include water-soluble polymers, such as polyethylene glycol, carboxymethyl cellulose, methyl cellulose, polyvinylpyrrolidone, polyvinyl alcohol, polyacrylic acid, and polyglycosyl ethyl methacrylate.

In addition, the first reagent and second reagent of the present disclosure may each include a chelating agent, such as EDTA, CyDTA, DTPA, EGTA, NTA, or NTP, a protein, such as bovine serum albumin, casein, or gelatin, a non-specific reaction inhibitor such as a protein degradation product, an amino acid, animal serum, an antibody, an antibody fragment, or a reducing agent, a stabilizer, such as a protein or a preservative, or an inorganic salt, such as sodium chloride, potassium chloride, or calcium chloride.

### <Affinity Particle and Pre-sensitization Particle>

The zeta potential of the affinity particle of the present disclosure is preferably -50 mV or more and -15 mV or less. When the zeta potential is -50 mV or more, an electrostatic repulsion force and an electrostatic attraction force between the affinity particles is appropriately balanced. Accordingly, an influence of an ionic component in the specimen is reduced, and hence more excellent measurement accuracy is obtained when the amount of change in absorbance per 1.0 ng/mL of the target substance is 0.0012 or more. In addition, when the zeta potential is -15 mV or less, the rate of particle aggregation is appropriately suppressed, and hence more excellent measurement accuracy is obtained in the case where the amount of change in absorbance per 1.0 ng/mL of the target substance is 0.0012 or more. In addition, excellent measurement accuracy is obtained even when the concentration of the target substance in the specimen is high. The zeta potential of the affinity particle is more preferably -40 mV or more and -15 mV or less.

The volume average particle diameter of the affinity particles of the present disclosure is preferably 200 nm or more and 500 nm or less. When the volume average particle diameter falls within the above-mentioned range, the particle number concentration of the particles falls within an appropriate range in the measurement method of the present disclosure. Accordingly, the action of the specimen component on the affinity particle becomes more uniform, and hence more excellent measurement accuracy is obtained. The volume average particle diameter is more preferably 250 nm or more and 400 nm or less.

Further, the affinity particle of the present disclosure is an affinity particle obtained by mixing two kinds of affinity particles having different volume average particle diameters, and a difference in volume average particle diameter between the two kinds of affinity particles is preferably 50 nm or more and 250 nm or less. The mixing of the two kinds of affinity particles having different volume average particle diameters enables the quantitative determination of the target substance for a wider range of target substance concentrations. At that time, when the difference in particle diameter falls within the above-mentioned range, the action of the specimen component on the affinity particle is less likely to be biased, and hence excellent measurement accuracy is obtained over a wide range of target substance concentrations.

In addition, the refractive index of the affinity particle of the present disclosure is preferably 1.600 or more and 1.800 or less. Further, the affinity particle is more preferably an affinity particle obtained by mixing two kinds of affinity particles having different refractive indices. In this case, a more excellent measurement range is obtained.

A conventionally known particle may be used as the pre-sensitization particle to be used in the present disclosure. For example, polystyrene, a styrene-butadiene copolymer, a styrene-styrenesulfonate copolymer, or a styrene-glycidyl methacrylate copolymer may be used. Of those, a particle containing a polymer including a repeating unit represented by the following formula (1) is preferred as the pre-sensitization particle: 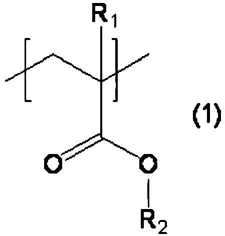 where R₁ represents a methyl group or a hydrogen atom, and R₂ represents a group having any one of an epoxy group, a hydroxy group, or a carboxy group.

When the pre-sensitization particle contains a polymer including the repeating unit represented by the formula (1), the surface of the affinity particle is easily hydrated, and the ionicity thereof is low, and hence the influence of a hydrophobic ion component is suppressed. As a result, higher measurement accuracy is obtained. The formula (1) represents more preferably a structure represented by the formula (1-A): 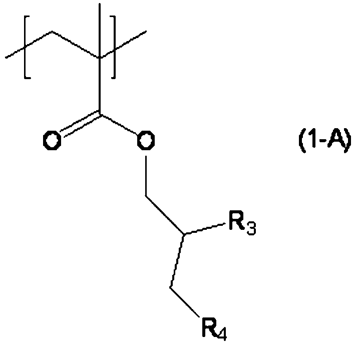 where at least one of R₃ or R₄ represents a hydroxy group, and the other thereof represents a hydroxy group or a group represented by the formula (1-B): 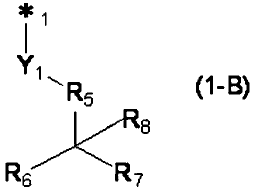 where R₅ represents a single bond or a methylene group, R₆, R₇, and R₈ are each selected from a hydrogen atom, a methyl group, a hydroxy group, a carboxy group, a hydroxymethyl group, and a carboxymethyl group, and at least one of R₆, R₇, or R₈ includes a hydroxy group or a carboxy group, Y₁ represents a sulfur atom or an imino group, and *₁ represents a bonding position with the structure represented by the formula (1-A).

Specific examples of the structure represented by the formula (1-A) are represented by the following formulae (1-A-1) to (1-A-12), but the present disclosure is not limited thereto.

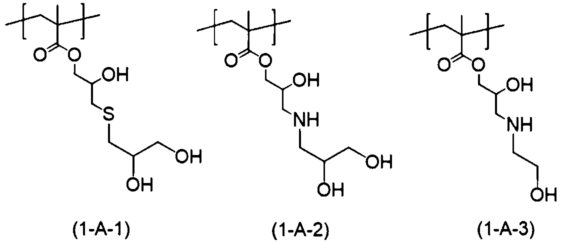

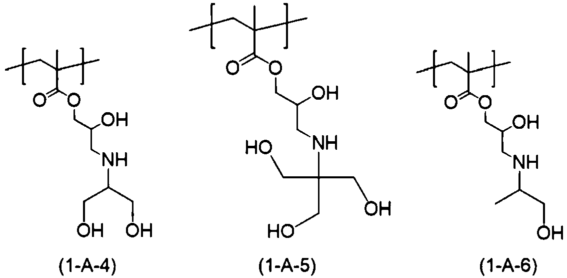

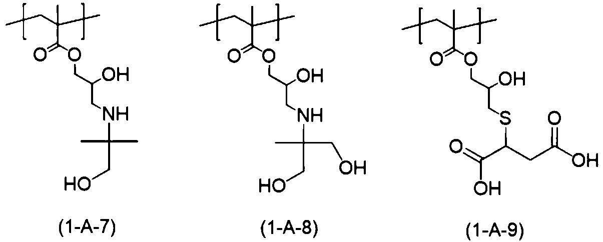

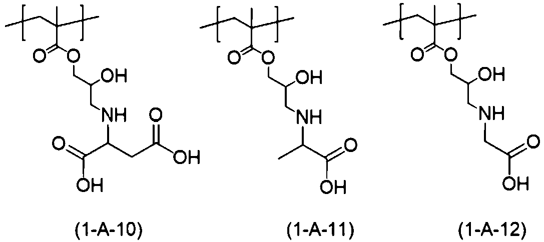

### <Ligand that specifically binds to Protein>

The ligand is a compound that specifically binds to a receptor of a specific target substance. A site where the ligand binds to the target substance is fixed, and has selectively or specifically high affinity. Examples thereof include an antigen and an antibody, an enzyme protein and a substrate therefor, a signal substance, which is typified by a hormone or a neurotransmitter, and a receptor therefor, a nucleic acid, and avidin and biotin, but the present disclosure is not limited thereto to the extent that the object of the present disclosure can be achieved. Specific examples of the ligand include an antigen, an antibody, an antigen-binding fragment (e.g., Fab, F(ab')₂, F(ab'), Fv, or scFv), a naturally derived nucleic acid, an artificial nucleic acid, an aptamer, a peptide aptamer, an oligopeptide, an enzyme, and a coenzyme.

In this embodiment, the ligand is preferably an antibody or an antigen. Of those, an antibody having an isoelectric point of 5.0 or more and 8.0 or less is more preferred. The affinity particle using an antibody having an isoelectric point that falls within the range has small charge on its surface because of the influence of the antibody. As a result, an interaction between the affinity particle and a cationic component in the specimen is reduced, and hence excellent measurement accuracy is obtained even when there are a large number of specimens. The isoelectric point may be measured by isoelectric focusing.

In the present disclosure, any known method may be adopted as a method of immobilizing the ligand on the pre-sensitization particle, and the ligand can be immobilized by being physically or chemically bonded to the pre-sensitization particle. For example, the ligand can be immobilized on the pre-sensitization particle by a covalent bond, a hydrogen bond, an ionic bond, electrostatic attraction, or a Van der Waals force. Examples of a method for the chemical bonding include a method involving utilizing a carbodiimide-mediated reaction or an NHS ester activation reaction, and a method of bonding a biotin-modified ligand to an avidin-bonded carboxy group.

The amount of the ligand with respect to 1.0 mg of the pre-sensitization particle is preferably 1.0 µg or more and 150.0 µg or less, more preferably 2.0 µg or more and 100.0 µg or less. Further, the affinity particle in the present disclosure is an affinity particle obtained by mixing two kinds of affinity particles, and it is still more preferred that the amounts of the ligand in the two kinds of affinity particles be different from each other.

In addition, the amount of the ligand in the second mixed solution is preferably 0.01 mg/mL or more and 2.00 mg/mL or less. When the amount of the ligand falls within the above-mentioned range, more excellent measurement accuracy is obtained in the case where the amount of change in absorbance per 1.0 ng/mL of the target substance is 0.0012 or more.

Examples of methods of measuring physical properties in the present disclosure are described below.

### <Method of measuring Viscosity>

The viscosity is measured with RE-85L (Toki Sangyo Co., Ltd.). 1.1 Milliliters of the sample was loaded into a measurement container, and its viscosity was measured with a cone rotor under the condition of a shear rate of 100 rpm. A measurement temperature was set to 25°C by connecting a circulating constant temperature bath.

### <Method of measuring Surface Tension>

The surface tension is measured with an automatic surface tensiometer DyneMaster (Kyowa Interface Science Co., Ltd.). The measurement is performed by a plate method using a platinum plate under a temperature condition of 25°C.

### <Method of measuring Electrical Conductivity>

The electrical conductivity is measured with an electrical conductivity meter AS710 (AS ONE Corporation). The measurement is performed under the conditions of a temperature of 23°C and a humidity of 50%.

### <Method of measuring Zeta Potential of Particle>

The zeta potential of the particle is measured with Zetasizer Nano ZS (Malvern Panalytical). The zeta potential in the present disclosure is measured under a state in which the particle is dispersed in a 0.01 N potassium hydroxide aqueous solution having a pH of 7.8 so that the concentration of the particle becomes 0.003 mass/vol%. With regard to measurement conditions, a temperature is 25°C, latex (n≈1.59) is selected as the refractive index of the particle, and pure water is selected as a solvent. The measurement is performed ten times, and the average value of the ten measured values is adopted as the zeta potential.

### <Method of measuring Volume Average Particle Diameter of Particles>

The volume average particle diameter of the particles is measured with Zetasizer Nano ZS (Malvern Panalytical). The volume average particle diameter in the present disclosure is measured under a state in which the particles are dispersed in ion-exchanged water so that the concentration of the particles becomes 0.003 mass/vol%. With regard to measurement conditions, a temperature is 25°C, latex (n≈1.59) is selected as the refractive index of each of the particles, and pure water is selected as a solvent. The measurement is performed ten times, and the average value of the ten measured values is adopted as the volume average particle diameter.

In addition, a difference in volume average particle diameter when two or more kinds of particles having different particle diameters are mixed may be determined by observation with a scanning electron microscope or the like. Specifically, 300 or more particles are photographed at a magnification of 50,000 times, and the particle diameters of the 300 particles are measured by a known image processing method. The volumes of the respective particles are determined from the measured particle diameters, and a volume distribution is prepared. A difference in particle diameter between the two kinds of particles may be determined by calculating the difference using the particle diameter at which a peak appears in the prepared volume distribution as the volume average particle diameter of the respective particles.

### <Method of measuring Refractive Index of Particle>

The refractive index of the particle may be measured with Abbemat (manufactured by Anton Paar). In the following Examples, specifically, the refractive index was measured under a state in which the particle was dispersed in a dispersion medium so that the concentration of the particle became 5 mass%. A temperature of 25°C and a measurement wavelength of 589.3 nm were selected as measurement conditions. The particle refractive index was calculated from the Lorentz-Lorenz equation by using the measured refractive index value, the specific gravity and refractive index of the dispersion medium, and the specific gravity of the particle.

### <Method of measuring Amount of Antibody with respect to Particle (Antibody Sensitization Amount of Affinity Particle)>

A method of measuring the antibody sensitization amount of the affinity particle in the present disclosure is described. The antibody sensitization amount with respect to the affinity particle was determined by protein quantification. Herein, the term "antibody sensitization amount with respect to the particle" means the amount of the bound or adsorbed antibody per 1 mg of the particle.

Specifically, first, 7 mL of the liquid A of Protein Assay BCA Kit (FUJIFILM Wako Pure Chemical Corporation) and 140 µL of the liquid B thereof were mixed, and the prepared liquid was adopted as a liquid AB. Next, 25 µL (particle concentration: 0.1 mass/vol%) of a dispersion of the affinity particle was added to 200 µL of the liquid AB, and the mixture was incubated at 60°C for 30 minutes. After that, the resultant solution was centrifuged at 4°C and 20,400×g for 5 minutes, and 200 µL of the supernatant was loaded into a 96-well microwell with a pipette. A standard sample, in which an antibody was mixed in a 10 mM HEPES buffer at an arbitrary concentration (five points within the concentration range of from 0 µg/mL to 200 µg/mL), was also loaded into a different location in the microwell in an amount of 200 µL, and the absorbance of the supernatant at 562 nm was collectively measured with a microplate reader, followed by the calculation of the antibody amount from the calibration curve of the standard sample. The antibody amount (µg/mg) with respect to the particle was determined by dividing the calculated antibody amount by the mass of the particle.

### [Examples]

The present disclosure is described in detail below by way of Examples, but the present disclosure is not limited to these Examples.

### (Synthesis of Particle 1)

The synthesis of a particle 1 includes the following steps 1 to 3.

### (Step 1)

71.75 Grams of styrene (St: Kishida Chemical Co., Ltd.), 1.30 g of divinylbenzene (DVB: Kishida Chemical Co., Ltd.), and 1,190.67 g of ion-exchanged water were weighed into a 2 L four-necked separable flask to prepare a mixed solution. Oxygen was removed from the inside of the four-necked separable flask by holding the mixed solution at 70°C under stirring at 200 rpm and performing a nitrogen flow at a flow rate of 200 mL/min. Next, a dissolved solution of 3.11 g of V-50 (FUJIFILM Wako Pure Chemical Corporation) dissolved in 50 g of ion-exchanged water, which had been separately prepared, was added to the mixed solution, and a polymerization reaction was performed for 48 hours to provide a dispersion containing a copolymer particle of St and DVB.

### (Step 2)

Next, 148.29 g of the above-mentioned dispersion diluted with ion-exchanged water so as to have a solid content concentration of 2.0 mass% was weighed into another four-necked separable flask. Next, 0.39 g of glycidyl methacrylate (GMA: Kishida Chemical Co., Ltd.) was added thereto. Oxygen was removed from the inside of the four-necked separable flask by holding the mixed solution at 70°C under stirring at 100 rpm and performing a nitrogen flow at a flow rate of 200 mL/min. Then, a dissolved solution of 0.018 g of V-50 dissolved in 1 g of ion-exchanged water, which had been separately prepared, was added to the mixed solution. A dispersion containing a St/DVB/GMA composite particle was obtained by continuing stirring for 17 hours.

### (Step 3)

Finally, an aqueous solution in which mercaptosuccinic acid (MSA: FUJIFILM Wako Pure Chemical Corporation) and 3-mercapto-1,2-propanediol (MPD: FUJIFILM Wako Pure Chemical Corporation) were dissolved, which had been prepared in advance, was added to the dispersion containing the St/DVB/GMA composite particle. At this time, the aqueous solution was prepared so that the molar ratio of 3-mercapto-1,2-propanediol to mercaptosuccinic acid was 6:4 (molar fraction), and the total number of moles of MSA and MPD was equal to the number of moles of glycidyl methacrylate. Subsequently, triethylamine (Kishida Chemical Co., Ltd.) was added thereto to adjust the pH to 10. Next, the temperature of the dispersion was increased to 70°C under stirring at 200 rpm, and the state was maintained for an additional 18 hours. Thus, a dispersion of a particle 1 was obtained. After that, the operation of separating the particle 1 from the dispersion with a centrifuge and redispersing the particle 1 in ion-exchanged water was repeated eight times to purify the particle 1, and finally, a particle 1 dispersion having a solid content concentration of 5.0 mass% was obtained. The volume average particle diameter of the resultant particle 1 was 400 nm.

### (Synthesis of Particle 2 to Particle 8)

A particle 2 to a particle 8 were each synthesized by the same experimental operation as that in the method of synthesizing the particle 1 except that, in the method of synthesizing the particle 1, the amount of styrene, the amount of DVB, the amount of V-50, and the stirring speed used in the step 1, the amount of GMA in the step 2, and the molar fractions of MSA and MPD in the step 3 were changed as shown in Table 1 (Tables 1-1 and 1-2). The physical properties of the resultant particle 1 to particle 8 are collectively shown in Table 1.

In each of the particles 1 to 8, a core particle included a styrene-divinylbenzene copolymer, and contained a polymer including a structural unit represented by the formula (1) on its surface. More specifically, the polymer included the structural unit represented by the formula (1) in which R₁ represented a methyl group, and R₂ was represented by the following formula (31), formula (32), formula (33), or formula (34): 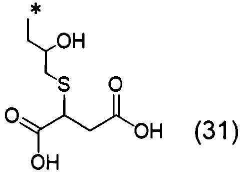 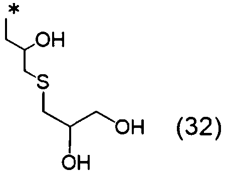 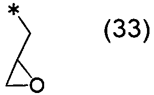 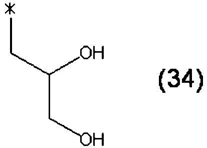 where * represents a bonding position with the structure represented by the formula (1).

**Table 1-1**

| | Synthesis conditions | | | | |
|---|---|---|---|---|---|
| | Step 1 | | | | Step 2 |
| | Amount of styrene (g) | Amount of DVB (g) | Amount of V-50 (g) | Stirring speed (rpm) | Amount of GMA (g) |
| Particle 1 | 71.75 | 1.30 | 3.11 | 200 | 0.39 |
| Particle 2 | 14.35 | 0.26 | 0.62 | 140 | 1.54 |
| Particle 3 | 46.64 | 0.85 | 2.02 | 140 | 0.39 |
| Particle 4 | 100.45 | 1.82 | 4.35 | 200 | 0.39 |
| Particle 5 | 71.75 | 1.30 | 3.11 | 200 | 0.39 |
| Particle 6 | 71.75 | 1.30 | 3.11 | 200 | 0.39 |
| Particle 7 | 71.75 | 1.30 | 3.11 | 200 | 0.39 |
| Particle 8 | 71.75 | 1.30 | 3.11 | 200 | 0.39 |

**Table 1-2**

| | Synthesis conditions | Physical properties | |
|---|---|---|---|
| | Step 3 | | |
| | MSA:MPD (molar fraction) | Volume average particle diameter (nm) | Zeta potential (mV) |
| Particle 1 | 6:4 | 400 | -40 |
| Particle 2 | 6:4 | 200 | -52 |
| Particle 3 | 6:4 | 300 | -38 |
| Particle 4 | 6:4 | 500 | -42 |
| Particle 5 | 4:6 | 410 | -31 |
| Particle 6 | 8:2 | 390 | -61 |
| Particle 7 | 3:7 | 420 | -27 |
| Particle 8 | 10:0 | 410 | -69 |

### (Preparation of Affinity Particle 1)

300 Microliters (3 mg in terms of particle solid content) of the dispersion of the particle 1 diluted with ion-exchanged water to a solid content concentration of 1.0 mass/vol% was aliquoted into a 1.5 mL microtube. 90 Microliters of a 5.0 mass% aqueous solution of 1-(3-dimethylaminopropyl)-3-ethylcarbodiimide hydrochloride and 90 µL of a 5.0 mass% aqueous solution of N-hydroxysulfosuccinimide sodium were added thereto. The mixture was stirred at room temperature for 30 minutes to provide a dispersion of a particle having an activated carboxy group (activated particle dispersion).

After centrifugal washing, 270 µL of phosphate-buffered saline (hereinafter referred to as "PBS") having a pH of 5.5 was added thereto, and the particle having an activated carboxy group was dispersed therein with an ultrasonic wave. 24 Microliters (0.12 mg in terms of antibody amount) of a 5.0 mg/mL dispersion of a mouse monoclonal anti-ferritin antibody (isoelectric point: 7.1) was added as a ligand thereto, and a test particle resulting from antibody sensitization on the particle was obtained by stirring at room temperature for 3 hours. After the centrifugal washing of the test particle, 500 µL of PBS was added thereto to provide an affinity particle 1.

### (Preparation of Affinity Particle 2 to Affinity Particle 10)

An affinity particle 2 to an affinity particle 10 were each prepared by the same experimental operation as that in the preparation of the affinity particle 1 except that, in the method of preparing the affinity particle 1, the kind of the particle, the kind of the antibody, and the addition amount of the antibody dispersion were changed as shown in Table 2.

**Table 2**

| | Particle No. | Kind of antibody | Addition amount of antibody dispersion (µL) | Amount of antibody per 1 mg of pre-sensitization particle (µg/mg) | Refractive index |
|---|---|---|---|---|---|
| Affinity particle 1 | 1 | Mouse monoclonal anti-ferritin antibody (isoelectric point: 7.1) | 24 | 40 | 1.705 |
| Affinity particle 2 | 2 | Mouse monoclonal anti-ferritin antibody (isoelectric point: 7.1) | 12 | 20 | 1.654 |
| Affinity particle 3 | 3 | Mouse monoclonal anti-ferritin antibody (isoelectric point: 7.1) | 24 | 40 | 1.705 |
| Affinity particle 4 | 4 | Mouse monoclonal anti-ferritin antibody (isoelectric point: 7.1) | 24 | 40 | 1.705 |
| Affinity particle 5 | 5 | Mouse monoclonal anti-ferritin antibody (isoelectric point: 7.1) | 24 | 40 | 1.705 |
| Affinity particle 6 | 6 | Mouse monoclonal anti-ferritin antibody (isoelectric point: 7.1) | 24 | 40 | 1.705 |
| Affinity particle 7 | 7 | Mouse monoclonal anti-ferritin antibody (isoelectric point: 7.1) | 24 | 40 | 1.705 |
| Affinity particle 8 | 8 | Mouse monoclonal anti-ferritin antibody (isoelectric point: 7.1) | 24 | 40 | 1.705 |
| Affinity particle 9 | 1 | Rabbit monoclonal anti-ferritin antibody (isoelectric point: 6.2) | 24 | 35 | 1.705 |
| Affinity particle 10 | 1 | Mouse monoclonal anti-ferritin antibody (isoelectric point: 7.9) | 24 | 40 | 1.705 |

### (Preparation of Second Reagent 1)

A second reagent 1 was prepared by using the prepared affinity particles 1 and 2. Specifically, the affinity particles 1 and 2 were each centrifuged, and re-dispersed in 500 µL of a buffer (HEPES buffer) in which 10 mM HEPES, 0.01 mass% polyoxyethylene nonylphenyl ether (Triton X-100: Kishida Chemical Co., Ltd.), and 10 mass% sucrose (viscosity modifier) were dissolved in ion-exchanged water. After that, the contents were mixed and diluted with the HEPES buffer so that the concentrations of the affinity particle 1 and the affinity particle 2 became 0.1 mass/vol% and 0.1 mass/vol%, respectively, and the total concentration was 0.2 mass/vol%. Thus, the second reagent 1 was obtained.

### (Preparation of Second Reagent 2 to Second Reagent 15)

A second reagent 2 to a second reagent 15 were each prepared by the same experimental operation as that in the preparation of the second reagent 1 except that, in the preparation of the second reagent 1, the kinds of the affinity particles, the concentrations of the affinity particles, and the composition of the viscosity modifier added to the HEPES buffer were changed as shown in Table 3 (Tables 3-1, 3-2 and 3-3). The physical properties of the resultant second reagent 1 to second reagent 15 are collectively shown in Table 3.

**Table 3-1**

| | Preparation conditions | |
|---|---|---|
| | Kind and concentration of affinity particle | |
| Second reagent 1 | Affinity particle 1 | : 0.1 mass/vol% |
| | Affinity particle 2 | : 0.1 mass/vol% |
| Second reagent 2 | Affinity particle 3 | : 0.1 mass/vol% |
| | Affinity particle 2 | : 0.1 mass/vol% |
| Second reagent 3 | Affinity particle 4 | : 0.1 mass/vol% |
| | Affinity particle 2 | : 0.1 mass/vol% |
| Second reagent 4 | Affinity particle 1 | : 0.05 mass/vol% |
| | Affinity particle 2 | : 0.05 mass/vol% |
| Second reagent 5 | Affinity particle 5 | : 0.1 mass/vol% |
| | Affinity particle 2 | : 0.1 mass/vol% |
| Second reagent 6 | Affinity particle 6 | : 0.1 mass/vol% |
| | Affinity particle 2 | : 0.1 mass/vol% |
| Second reagent 7 | Affinity particle 7 | : 0.1 mass/vol% |
| | Affinity particle 2 | : 0.1 mass/vol% |
| Second reagent 8 | Affinity particle 8 | : 0.1 mass/vol% |
| | Affinity particle 2 | : 0.1 mass/vol% |
| Second reagent 9 | Affinity particle 1 | : 0.1 mass/vol% |
| | Affinity particle 2 | : 0.1 mass/vol% |
| Second reagent 10 | Affinity particle 1 | : 0.1 mass/vol% |
| | Affinity particle 2 | : 0.1 mass/vol% |
| Second reagent 11 | Affinity particle 1 | : 0.1 mass/vol% |
| | Affinity particle 2 | : 0.1 mass/vol% |
| Second reagent 12 | Affinity particle 9 | : 0.1 mass/vol% |
| | Affinity particle 2 | : 0.1 mass/vol% |
| Second reagent 13 | Affinity particle 10 | : 0.1 mass/vol% |
| | Affinity particle 2 | : 0.1 mass/vol% |
| Second reagent 14 | Affinity particle 1 | : 0.1 mass/vol% |
| | Affinity particle 2 | : 0.1 mass/vol% |
| Second reagent 15 | Affinity particle 3 | : 0.1 mass/vol% |
| | Affinity particle 2 | : 0.1 mass/vol% |

**Table 3-2**

| | | |
|---|---|---|
| | | |

| | Viscosity modifier to be added to HEPES buffer | |
|---|---|---|
| Second reagent 1 | Sucrose | : 10 mass% |
| Second reagent 2 | Sucrose | : 10 mass% |
| Second reagent 3 | Sucrose | : 10 mass% |
| Second reagent 4 | Sucrose | : 10 mass% |
| Second reagent 5 | Sucrose | : 10 mass% |
| Second reagent 6 | Sucrose | : 10 mass% |
| Second reagent 7 | Sucrose | : 10 mass% |
| Second reagent 8 | Sucrose | : 10 mass% |
| Second reagent 9 | Sucrose | : 5 mass% |
| Second reagent 10 | Sucrose | : 10 mass% |
| | Polyethylene glycol (molecular weight: 20,000) | : 1 mass% |
| Second reagent 11 | - | |
| Second reagent 12 | Sucrose | : 10 mass% |
| Second reagent 13 | Sucrose | : 10 mass% |
| Second reagent 14 | Sucrose | : 10 mass% |
| Second reagent 15 | - | |

**Table 3-3**

| | Physical properties | | |
|---|---|---|---|
| | Volume average particle diameter (nm) | Difference in volume average particle diameter between two kinds of particles (nm) | Zeta potential (mV) |
| Second reagent 1 | 300 | 200 | -25 |
| Second reagent 2 | 250 | 100 | -29 |
| Second reagent 3 | 350 | 300 | -33 |
| Second reagent 4 | 300 | 200 | -25 |
| Second reagent 5 | 310 | 210 | -17 |
| Second reagent 6 | 295 | 190 | -45 |
| Second reagent 7 | 315 | 220 | -14 |
| Second reagent 8 | 310 | 210 | -55 |
| Second reagent 9 | 300 | 200 | -25 |
| Second reagent 10 | 300 | 200 | -25 |
| Second reagent 11 | 300 | 200 | -25 |
| Second reagent 12 | 300 | 200 | -31 |
| Second reagent 13 | 300 | 200 | -21 |
| Second reagent 14 | 300 | 200 | -25 |
| Second reagent 15 | 250 | 100 | -29 |

### (Preparation of First Reagent 1 to First Reagent 8)

As shown in Table 4, liquids in which the respective materials were mixed in predetermined amounts in ion-exchanged water were used as a first reagent 1 to a first reagent 8. The physical properties of the resultant first reagent 1 to first reagent 8 are collectively shown in Table 4.

**Table 4**

| | | Physical properties of first reagent | |
|---|---|---|---|
| | Material kind and addition amount | Surface tension (mN/m) | Electric conductivity (mS/cm) |
| First reagent 1 | 50 mM HEPES | 35 | 18.6 |
| | 0.05 mass% TritonX-100 | | |
| | 1.0 mass% NaCl | | |
| First reagent 2 | 50 mM HEPES | 30 | 18.6 |
| | 0.1 mass% TritonX-100 | | |
| | 1.0 mass% NaCl | | |
| First reagent 3 | 50 mM HEPES | 48 | 18.6 |
| | 0.005 mass% TritonX-100 | | |
| | 1.0 mass% NaCl | | |
| First reagent 4 | 50 mM HEPES | 55 | 18.6 |
| | 0.001 mass% TritonX-100 | | |
| | 1.0 mass% NaCl | | |
| First reagent 5 | 50 mM HEPES | 35 | 0.9 |
| | 0.05 mass% TritonX-100 | | |
| First reagent 6 | 50 mM HEPES | 35 | 67.9 |
| | 0.05 mass% TritonX-100 | | |
| | 3.8 mass% NaCl | | |
| First reagent 7 | 10 mM HEPES | 35 | 0.4 |
| | 0.05 mass% TritonX-100 | | |
| First reagent 8 | 50 mM HEPES | 35 | 75.0 |
| | 0.05 mass% TritonX-100 | | |
| | 4.2 mass% NaCl | | |

### (Example 1: Measurement of Amount of Change in Absorbance)

A mixed solution was prepared by mixing 15 µL of a specimen and 60 µL of the first reagent 1, and the mixture was kept warm for 290 seconds under the condition of 37°C. Next, 30 µL of the second reagent 1 was mixed with the above-mentioned mixed solution, and the mixture was stirred, followed by the measurement of its absorbance 40 seconds after the stirring. Further, the mixed solution was allowed to stand at 37°C for 253 seconds, and then its absorbance was measured again. The difference of the absorbance from the absorbance 40 seconds after the stirring was defined as the amount of change in absorbance. A spectrophotometer BIOSPECTROMETER (Eppendorf) was used in the absorbance measurement, and a measurement wavelength was set to 572 nm. An optical path length was 1 cm. The kind of the used specimen is described in a description of each evaluation item to be described later.

### (Method of measuring Amount of Change in Absorbance per 1.0 ng/mL of Ferritin)

In the measurement of the amount of change in absorbance, a difference between the average value of the amounts of change in absorbance when the measurement was performed 20 times by using PBS (free of ferritin) as a specimen and the average value of the amounts of change in absorbance when the measurement was performed 20 times by using, as a specimen, a sample A containing 1.7 ng/mL of ferritin was calculated. A value obtained by subtracting the average amount of change in absorbance when PBS was used from the average amount of change in absorbance when the sample A was used, and dividing the resultant value by 1.7 was defined as the amount of change in absorbance per 1.0 ng/mL.

### (Method of evaluating Measurement Accuracy when Concentration of Target Substance is low (Low-concentration Measurement Accuracy))

In the measurement of the amount of change in absorbance, the average value of a standard deviation (hereinafter referred to as "SD") when the measurement was performed 20 times by using PBS as a specimen and a SD when the measurement was performed 20 times by using, as a specimen, the sample A containing 1.7 ng/mL of ferritin was calculated. The resultant average SD was evaluated in accordance with the following criteria.
A: The SD was less than 0.00030.
B: The SD was 0.00030 or more and less than 0.00040.
C: The SD was 0.00040 or more and less than 0.00050.
D: The SD was 0.00050 or more and less than 0.00070.
E: The SD was 0.00070 or more and less than 0.00100.
F: The SD was 0.00100 or more.

### (Method of evaluating Lowest Detection Sensitivity)

In the measurement of the amount of change in absorbance, measurement was performed by using a specimen obtained by diluting a sample B containing 12.5 ng/mL of ferritin with PBS. The measurement is performed 20 times for each of specimens prepared so that their ferritin concentrations become 0.0 ng/mL (PBS), 1.0 ng/mL, 1.5 ng/mL, 2.0 ng/mL, 2.5 ng/mL, and 3.0 ng/mL. Next, an average amount of change in absorbance and a SD are calculated for each specimen. When a value A obtained by adding a value that was 2.6 times as large as the SD to the average amount of change in absorbance at 0.0 ng/mL, and a value B obtained by subtracting a value that was 2.6 times as large as the SD from the average amount of change in absorbance of each specimen satisfied A<B, it was determined that the concentration was able to be identified. The lowest ferritin concentration that was able to be identified was evaluated in accordance with the following criteria.
A: A concentration of 1.0 ng/mL can be identified.
B: A concentration of 1.5 ng/mL can be identified.
C: A concentration of 2.0 ng/mL can be identified.
D: A concentration of 2.5 ng/mL can be identified.
E: A concentration of 3.0 ng/mL can be identified.
F: A concentration of 3.0 ng/mL cannot be identified

### (Method of evaluating Measurement Accuracy when Concentration of Target Substance is high (High-concentration Measurement Accuracy))

In the measurement of the amount of change in absorbance, a coefficient of variation (CV) when the measurement was performed 5 times by using a sample C containing 550.0 ng/mL of ferritin was calculated. The CV is a value obtained by dividing the SD by the average value of the amounts of change in absorbance. The resultant CV was evaluated in accordance with the following criteria.
A: The CV was less than 0.008.
B: The CV was 0.008 or more and less than 0.010.
C: The CV was 0.010 or more and less than 0.015.
D: The CV was 0.015 or more and less than 0.020.
E: The CV was 0.020 or more and less than 0.030.
F: The CV was 0.030 or more.

### (Example 1 to Example 5 and Comparative Example 1 to Comparative Example 4)

An amount of change in absorbance was measured and various evaluations were performed by the same experimental operation as that of the measurement method for the amount of change in absorbance in Example 1 except that the amount of the specimen, the kind and amount of the first reagent, the kind and amount of the second reagent, and the measurement wavelength in the measurement of the amount of change in absorbance in Example 1 were changed as shown in Table 5 (Tables 5-1 and 5-2). The physical properties and evaluation results under the respective measurement conditions are collectively shown in Table 6 (Tables 6-1 and 6-2).

**Table 5-1**

| | Sample amount (µL) | First reagent | | Second reagent | |
|---|---|---|---|---|---|
| | | Kind | Amount (µL) | Kind | Amount (µL) |
| Example 1 | 15 | 1 | 60 | 1 | 30 |
| Example 2 | 8 | 1 | 67 | 1 | 30 |
| Example 3 | 25 | 1 | 50 | 1 | 30 |
| Example 4 | 15 | 1 | 60 | 2 | 30 |
| Example 5 | 15 | 1 | 60 | 3 | 30 |
| Comparative Example 1 | 10 | 1 | 65 | 2 | 30 |
| Comparative Example 2 | 5 | 1 | 70 | 2 | 30 |
| Comparative Example 3 | 20 | 1 | 55 | 4 | 30 |
| Comparative Example 4 | 35 | 1 | 40 | 1 | 30 |

**Table 5-2**

| | Measurement wavelength (nm) | Second mixing step | | Amount of change in absorbance per 1 ng/MI |
|---|---|---|---|---|
| | | Sample concentration (vol%) | Mass ratio of particles to sample | |
| Example 1 | 572 | 14.3 | 1:250 | 0.0025 |
| Example 2 | 572 | 7.6 | 1:133 | 0.0020 |
| Example 3 | 572 | 23.8 | 1:417 | 0.0028 |
| Example 4 | 572 | 14.3 | 1:250 | 0.0014 |
| Example 5 | 572 | 14.3 | 1:250 | 0.0045 |
| Comparative Example 1 | 572 | 9.5 | 1:167 | 0.0011 |
| Comparative Example 2 | 572 | 4.8 | 1:83 | 0.0006 |
| Comparative Example 3 | 572 | 19.1 | 1:667 | 0.0023 |
| Comparative Example 4 | 572 | 33.3 | 1:583 | 0.0052 |

**Table 6-1**

| | First reagent | | Second reagent (particle physical properties) | | |
|---|---|---|---|---|---|
| | Surface tension (mN/m) | Electric conductivity (mS/cm) | Zeta potential (mV) | Volume average particle diameter (nm) | Difference in particle diameter between two kinds of particles (nm) |
| Example 1 | 35 | 18.6 | -25 | 300 | 200 |
| Example 2 | 35 | 18.6 | -25 | 300 | 200 |
| Example 3 | 35 | 18.6 | -25 | 300 | 200 |
| Example 4 | 35 | 18.6 | -29 | 250 | 100 |
| Example 5 | 35 | 18.6 | -33 | 350 | 300 |
| Comparative Example 1 | 35 | 18.6 | -29 | 250 | 100 |
| Comparative Example 2 | 35 | 18.6 | -29 | 250 | 100 |
| Comparative Example 3 | 35 | 18.6 | -25 | 300 | 200 |
| Comparative Example 4 | 35 | 18.6 | -25 | 300 | 200 |

**Table 6-2**

| | Second mixing step | | Evaluation result | | |
|---|---|---|---|---|---|
| | Absorbance after 40 seconds | Viscosity (mPa·s) | Low concentration accuracy | Lowest detection sensitivity | High concentration accuracy |
| Example 1 | 1.6 | 1.03 | A | A | A |
| Example 2 | 1.6 | 1.03 | A | A | A |
| Example 3 | 1.6 | 1.03 | A | A | B |
| Example 4 | 1.1 | 1.03 | B | C | A |
| Example 5 | 2.0 | 1.03 | A | A | C |
| Comparative Example 1 | 1.6 | 1.03 | C | E | A |
| Comparative Example 2 | 1.6 | 1.03 | A | F | A |
| Comparative Example 3 | 0.8 | 1.03 | E | D | A |
| Comparative Example 4 | 1.6 | 1.03 | F | D | D |

Those results show that the measurement method satisfying the provisions of the present disclosure can detect the target substance even when the concentration of the target substance is 1.5 ng/mL or less, and hence the method is extremely excellent in lowest detection sensitivity. Meanwhile, it is found that when the measurement method does not satisfy the provisions of the present disclosure, even in the case where the amount of change in absorbance per 1.0 ng/mL of the target substance is 0.0012 or more, its lowest detection sensitivity is poor.

### (Example 6 to Example 26)

An amount of change in absorbance was measured and various evaluations were performed by the same experimental operation as that of the measurement method for the amount of change in absorbance in Example 1 except that the amount of the specimen, the kind and amount of the first reagent, the kind and amount of the second reagent, and the measurement wavelength in the measurement of the amount of change in absorbance in Example 1 were changed as shown in Table 7 (Tables 7-1 and 7-2). The physical properties and evaluation results under the respective measurement conditions are collectively shown in Table 8 (Tables 8-1 and 8-2).

**Table 7-1**

| | Sample amount (µL) | First reagent | | Second reagent | |
|---|---|---|---|---|---|
| | | Kind | Amount (µL) | Kind | Amount (µL) |
| Example 6 | 15 | 1 | 60 | 1 | 30 |
| Example 7 | 15 | 1 | 60 | 1 | 30 |
| Example 8 | 15 | 1 | 60 | 1 | 30 |
| Example 9 | 15 | 1 | 60 | 1 | 30 |
| Example 10 | 15 | 1 | 60 | 5 | 30 |
| Example 11 | 15 | 1 | 60 | 6 | 30 |
| Example 12 | 15 | 1 | 60 | 7 | 30 |
| Example 13 | 15 | 1 | 60 | 8 | 30 |
| Example 14 | 15 | 2 | 60 | 1 | 30 |
| Example 15 | 15 | 3 | 60 | 1 | 30 |
| Example 16 | 15 | 4 | 60 | 1 | 30 |
| Example 17 | 15 | 1 | 60 | 9 | 30 |
| Example 18 | 15 | 1 | 60 | 10 | 30 |
| Example 19 | 15 | 1 | 60 | 11 | 30 |
| Example 20 | 15 | 5 | 60 | 1 | 30 |
| Example 21 | 15 | 6 | 60 | 1 | 30 |
| Example 22 | 15 | 7 | 60 | 1 | 30 |
| Example 23 | 15 | 8 | 60 | 1 | 30 |
| Example 24 | 15 | 1 | 60 | 12 | 30 |
| Example 25 | 15 | 1 | 60 | 13 | 30 |
| Example 26 | 15 | 1 | 60 | 14 | 30 |

**Table 7-2**

| | Measurement wavelength (nm) | Second mixing step | | Amount of change in absorbance per 1 ng/mL |
|---|---|---|---|---|
| | | Sample concentration (vol%) | Mass ratio of particles to sample | |
| Example 6 | 500 | 14.3 | 1:250 | 0.0032 |
| Example 7 | 748 | 14.3 | 1:250 | 0.0020 |
| Example 8 | 476 | 14.3 | 1:250 | 0.0033 |
| Example 9 | 804 | 14.3 | 1:250 | 0.0016 |
| Example 10 | 572 | 14.3 | 1:250 | 0.0025 |
| Example 11 | 572 | 14.3 | 1:250 | 0.0020 |
| Example 12 | 572 | 14.3 | 1:250 | 0.0028 |
| Example 13 | 572 | 14.3 | 1:250 | 0.0017 |
| Example 14 | 572 | 14.3 | 1:250 | 0.0022 |
| Example 15 | 572 | 14.3 | 1:250 | 0.0027 |
| Example 16 | 572 | 14.3 | 1:250 | 0.0022 |
| Example 17 | 572 | 14.3 | 1:250 | 0.0025 |
| Example 18 | 572 | 14.3 | 1:250 | 0.0021 |
| Example 19 | 572 | 14.3 | 1:250 | 0.0025 |
| Example 20 | 572 | 14.3 | 1:250 | 0.0025 |
| Example 21 | 572 | 14.3 | 1:250 | 0.0020 |
| Example 22 | 572 | 14.3 | 1:250 | 0.0025 |
| Example 23 | 572 | 14.3 | 1:250 | 0.0019 |
| Example 24 | 572 | 14.3 | 1:250 | 0.0021 |
| Example 25 | 572 | 14.3 | 1:250 | 0.0024 |
| Example 26 | 572 | 14.3 | 1:250 | 0.0025 |

**Table 8-1**

| | First Reagent | | Second Reagent (particle physical properties) | | |
|---|---|---|---|---|---|
| | Surface tension (mN/m) | Electric conductivity (mS/cm) | Zeta potential (mV) | Volume average particle diameter (nm) | Difference in particle diameter between two kinds of particles (nm) |
| Example 6 | 35 | 18.6 | -25 | 300 | 200 |
| Example 7 | 35 | 18.6 | -25 | 300 | 200 |
| Example 8 | 35 | 18.6 | -25 | 300 | 200 |
| Example 9 | 35 | 18.6 | -25 | 300 | 200 |
| Example 10 | 35 | 18.6 | -17 | 310 | 210 |
| Example 11 | 35 | 18.6 | -45 | 295 | 190 |
| Example 12 | 35 | 18.6 | -14 | 315 | 220 |
| Example 13 | 35 | 18.6 | -55 | 310 | 210 |
| Example 14 | 30 | 18.6 | -25 | 300 | 200 |
| Example 15 | 48 | 18.6 | -25 | 300 | 200 |
| Example 16 | 55 | 18.6 | -25 | 300 | 200 |
| Example 17 | 35 | 18.6 | -25 | 300 | 200 |
| Example 18 | 35 | 18.6 | -25 | 300 | 200 |
| Example 19 | 35 | 18.6 | -25 | 300 | 200 |
| Example 20 | 35 | 0.9 | -25 | 300 | 200 |
| Example 21 | 35 | 67.9 | -25 | 300 | 200 |
| Example 22 | 35 | 0.4 | -25 | 300 | 200 |
| Example 23 | 35 | 75.0 | -25 | 300 | 200 |
| Example 24 | 35 | 18.6 | -31 | 300 | 200 |
| Example 25 | 35 | 18.6 | -21 | 300 | 200 |
| Example 26 | 35 | 18.6 | -25 | 300 | 200 |

**Table 8-2**

| | Second mixing step | | Evaluation result | | |
|---|---|---|---|---|---|
| | Absorbance after 40 seconds | Viscosity (mPa·s) | Low concentration accuracy | Lowest detection sensitivity | High concentration accuracy |
| Example 6 | 1.6 | 1.03 | A | A | B |
| Example 7 | 1.6 | 1.03 | A | A | A |
| Example 8 | 1.6 | 1.03 | B | A | C |
| Example 9 | 1.6 | 1.03 | A | B | A |
| Example 10 | 1.6 | 1.03 | B | A | B |
| Example 11 | 1.6 | 1.03 | B | B | B |
| Example 12 | 1.6 | 1.03 | C | B | C |
| Example 13 | 1.6 | 1.03 | C | C | C |
| Example 14 | 1.6 | 1.03 | A | A | A |
| Example 15 | 1.6 | 1.03 | B | A | A |
| Example 16 | 1.6 | 1.03 | C | C | A |
| Example 17 | 1.6 | 0.97 | B | A | B |
| Example 18 | 1.6 | 1.30 | B | A | A |
| Example 19 | 1.6 | 0.91 | C | B | C |
| Example 20 | 1.6 | 1.03 | B | A | A |
| Example 21 | 1.6 | 1.03 | B | B | A |
| Example 22 | 1.6 | 1.03 | C | B | A |
| Example 23 | 1.6 | 1.03 | C | C | A |
| Example 24 | 1.6 | 1.03 | A | A | A |
| Example 25 | 1.6 | 1.03 | A | A | A |
| Example 26 | 1.6 | 1.05 | A | A | A |

Those results show that the measurement method satisfying the provisions of the present disclosure is excellent in low-concentration measurement accuracy, lowest detection sensitivity, and high-concentration measurement accuracy, and that further excellent performance is obtained by satisfying the provisions of a preferred embodiment of the present disclosure.

### (Comparative Example 5 to Comparative Example 7)

An amount of change in absorbance was measured and various evaluations were performed by the same experimental operation as that of the measurement method for the amount of change in absorbance in Example 1 except that the amount of the specimen, the kind and amount of the first reagent, the kind and amount of the second reagent, and the measurement wavelength in the measurement of the amount of change in absorbance in Example 1 were changed as shown in Table 9 (Tables 9-1 and 9-2). The physical properties and evaluation results under the respective measurement conditions are collectively shown in Table 10 (Tables 10-1 and 10-2).

**Table 9-1**

| | Sample amount (µL) | First reagent | | Second reagent | |
|---|---|---|---|---|---|
| | | Kind | Amount (µL) | Kind | Amount (µL) |
| Comparative Example 5 | 10 | 1 | 65 | 2 | 30 |
| Comparative Example 6 | 10 | 1 | 65 | 15 | 30 |
| Comparative Example 7 | 10 | 7 | 65 | 2 | 30 |

**Table 9-2**

| | Measurement wavelength (nm) | Second mixing step | | Amount of change in absorbance per 1 ng/mL |
|---|---|---|---|---|
| | | Sample concentration (vol%) | Mass ratio of particles to sample | |
| Comparative Example 5 | 572 | 9.5 | 1:167 | 0.0011 |
| Comparative Example 6 | 572 | 9.5 | 1:167 | 0.0011 |
| Comparative Example 7 | 572 | 9.5 | 1:167 | 0.0011 |

**Table 10-1**

| | First reagent | | Second reagent (particle physical properties) | | |
|---|---|---|---|---|---|
| | Surface tension (mN/m) | Electric conductivity (mS/cm) | Zeta potential (mV) | Volume average particle diameter (nm) | Difference in particle diameter between two kinds of particles (nm) |
| Comparative Example 5 | 55 | 18.6 | -29 | 250 | 100 |
| Comparative Example 6 | 35 | 18.6 | -29 | 250 | 100 |
| Comparative Example 7 | 35 | 0.4 | -29 | 250 | 100 |

**Table 10-2**

| | Second mixing step | | Evaluation result | | |
|---|---|---|---|---|---|
| | Absorbance after 40 seconds | Viscosity (mPa·s) | Low concentratio n accuracy | Lowest detection sensitivity | High concentratio n accuracy |
| Comparative Example 5 | 1.6 | 1.03 | C | E | A |
| Comparative Example 6 | 1.6 | 0.91 | C | E | A |
| Comparative Example 7 | 1.6 | 1.03 | C | E | A |

Those results show that the measurement method that does not satisfy the provisions of the present disclosure is not effective even when the provisions of a preferred embodiment of the present disclosure are satisfied.

Ferritin has been described as an example of a target substance that can be used in the present disclosure. A protein in which a plurality of subunits are associated like ferritin has a plurality of reaction sites with an antibody, and hence it is conceivable that a plurality of particles may be involved to form non-uniform aggregation in measurement of immunoturbidimetry using a particle. From this viewpoint, it may be said that the measurement method of the present disclosure is intended for a substance having a plurality of reaction sites for a ligand specifically binding to a target substance.

Various embodiments have been described in detail above but it will be understood that the present disclosure is not limited to these embodiments and encompasses all modifications, variants, alternatives and equivalents falling within the scope of the appended claims.

## Claims

1. A measurement method for measuring a target substance in a specimen,
the target substance being a protein having a molecular weight of 150 kDa or more and 500 kDa or less, which is incorporated into the specimen at a concentration of 0.8 ng/mL or more and 2.0 ng/mL or less,
the measurement method comprising:
a first mixing step of mixing the specimen and a first reagent to provide a first mixed solution;
a second mixing step of mixing the first mixed solution and a second reagent, which includes a particle having immobilized thereon a ligand that specifically binds to the target substance, to provide a second mixed solution; and
a measurement step of measuring an absorbance of the second mixed solution,
wherein a concentration of the specimen in the second mixed solution is 4.0 vol% or more and 30.0 vol% or less,
wherein a mass ratio of the particle to the specimen in the second mixed solution is 1:500 or more and 1:50 or less, and
wherein the measurement method includes measuring the specimen in which an amount of change in absorbance per 1.0 ng/mL of the target substance in the measurement step is 0.0012 or more and less than 0.0100.

2. The measurement method according to claim 1, wherein the amount of change in absorbance per 1.0 ng/mL of the target substance in the measurement step is 0.0020 or more and less than 0.0100.

3. The measurement method according to claim 1 or 2, wherein a specimen including the target substance at a concentration of 1.0 ng/mL or more and 1.5 ng/mL or less is measured.

4. The measurement method according to any one of claims 1 to 3, wherein the specimen is selected from the group consisting of blood, serum, and plasma each including the target substance.

5. The measurement method according to any one of claims 1 to 4, wherein the concentration of the specimen in the second mixed solution is 7.0 vol% or more and 30.0 vol% or less.

6. The measurement method according to any one of claims 1 to 5, wherein a measurement wavelength of the absorbance includes 500 nm or more and 750 nm or less.

7. The measurement method according to any one of claims 1 to 6, wherein the absorbance 40 seconds after the mixing in the second mixing step is 0.90 or more and 2.00 or less.

8. The measurement method according to any one of claims 1 to 7, wherein a zeta potential of the particle is -50 mV or more and -15 mV or less.

9. The measurement method according to any one of claims 1 to 8, wherein a surface tension of the first reagent is 20 mN/m or more and 50 mN/m or less.

10. The measurement method according to any one of claims 1 to 9, wherein a viscosity of the second mixed solution is 0.95 mPa·s or more and 1.40 mPa·s or less.

11. The measurement method according to any one of claims 1 to 10, wherein an electrical conductivity of the first reagent is 0.5 mS/cm or more and 70.0 mS/cm or less.

12. The measurement method according to any one of claims 1 to 11, wherein a volume average particle diameter of the particles is 200 nm or more and 500 nm or less.

13. The measurement method according to any one of claims 1 to 12, wherein the particle is a particle obtained by mixing two kinds of particles having different volume average particle diameters, and a difference in volume average particle diameter between the two kinds of particles is 250 nm or less.

14. The measurement method according to any one of claims 1 to 13, wherein the target substance is a protein having a molecular weight of 300 kDa or more and 600 kDa or less.

15. The measurement method according to claim 14, wherein the target substance is ferritin.

16. The measurement method according to any one of claims 1 to 15, wherein the ligand that specifically binds to the target substance is an antibody having an isoelectric point of 5.0 or more and 8.0 or less.

17. The measurement method according to any one of claims 1 to 16, wherein the first reagent and the second reagent each include at least one of a sugar or a sugar alcohol.

18. The measurement method according to any one of claims 1 to 17, wherein the first reagent and the second reagent each include one or more surfactants selected from the group consisting of a sorbitan fatty acid ester, a polyoxyethylene alkyl ether, and a polyoxyethylene phenyl ether.

19. The measurement method according to any one of claims 1 to 18, wherein the particle contains a polymer having a structure represented by the following formula (1): 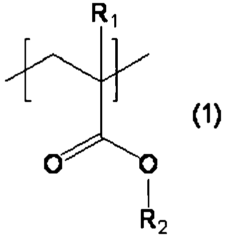 where R₁ represents a methyl group or a hydrogen atom, and R₂ represents a group having any one of an epoxy group, a hydroxy group, or a carboxy group.

20. The measurement method according to claim 19, wherein the formula (1) represents a structure represented by the following formula (1-A): 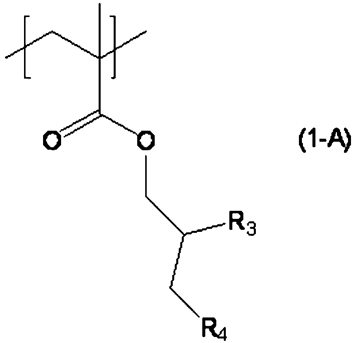 where at least one of R₃ or R₄ represents a hydroxy group, and another thereof represents a hydroxy group or a group represented by the following formula (1-B): 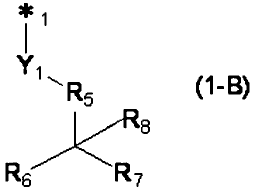 where R₅ represents a single bond or a methylene group, R₆, R₇, and R₈ are each selected from a hydrogen atom, a methyl group, a hydroxy group, a carboxy group, a hydroxymethyl group, and a carboxymethyl group, and at least one of R₆, R₇, or R₈ includes a hydroxy group or a carboxy group, Y₁ represents a sulfur atom or an imino group, and *₁ represents a bonding position with the structure represented by the formula (1-A).

21. The measurement method according to any one of claims 1 to 20, wherein the target substance is a substance having a plurality of reaction sites for the ligand that specifically binds to the target substance.

22. A reagent for measuring a target substance in a specimen for use in the measurement method of any one of claims 1 to 21,
wherein the target substance is a protein having a molecular weight of 150 kDa or more and 500 kDa or less, which is incorporated into the specimen at a concentration of 0.8 ng/mL or more and 2.0 ng/mL or less,
wherein the reagent comprises a first reagent and a second reagent including a particle having immobilized thereon a ligand that specifically binds to the target substance, and
wherein in a case where ΔOD1a represents a difference "absorbance B-absorbance A" between an absorbance A at a time point of 35 seconds or more and 45 seconds or less after addition of the second reagent and stirring, and an absorbance B at a time point of 290 seconds or more and 300 seconds or less thereafter when a first mixed solution is prepared by mixing 15 µL of the specimen, which includes 1.0 ng/mL of the target substance, 50 mM of N-2-hydroxyethylpiperazine-N'-2-ethanesulfonic acid, and ultrapure water, with 60 µL of the first reagent under a condition of 37°C, and 30 µL of the second reagent is added to the first mixed solution and the mixture is stirred at a time point after a lapse of 280 seconds or more and 300 seconds or less from the mixing, and ΔOD0a represents a difference "absorbance D-absorbance C" between an absorbance C at a time point of 35 seconds or more and 45 seconds or less after addition of the second reagent and stirring, and an absorbance D at a time point of 290 seconds or more and 300 seconds or less thereafter when the first mixed solution is prepared by mixing 15 µL of the specimen, which includes 50 mM of N-2-hydroxyethylpiperazine-N'-2-ethanesulfonic acid and ultrapure water, with 60 µL of the first reagent under the condition of 37°C, and 30 µL of the second reagent is added to the first mixed solution and the mixture is stirred at a time point after a lapse of 280 seconds or more and 300 seconds or less from the mixing, a difference "ΔOD1a-ΔODOa" is 0.0012 or more when a measurement wavelength of each of the absorbances is 572 nm.
